# EUROPÄISCHE PATENTSCHRIFT

(11) **EP 0 809 111 B1**
(45) Veröffentlichungstag und Bekanntmachung des Hinweises auf die Patenterteilung: **24.07.2002**
(21) Anmeldenummer: 97108401.7
(22) Anmeldetag: 23.05.1997
(51) Int. Cl.: G01N 33/58, G01N 33/543, C07K 16/46

(54) **Bestimmungsverfahren mit oligomerisierten Rezeptoren**
ANALYZING METHOD UTILIZING OLIGOMERIC RECEPTORS
PROCEDE D'ANALYSE UTILISANT DES RECEPTEURS OLIGOMERES

(30) Priorität: 24.05.1996 DE 19621133
(43) Veröffentlichungstag der Anmeldung: 26.11.1997
(73) Patentinhaber: Roche Diagnostics GmbH, 68305 Mannheim (DE)
(72) Erfinder: Neumann, Ulrich, Dr., 82362 Weilheim (DE); Lenz, Helmut, Dr., 82327 Tutzing (DE); Franken, Norbert, Dr., 82319 Starnberg (DE)
(74) Vertreter: Weiss, Wolfgang, Dipl.-Chem. Dr.

(56) Entgegenhaltungen:
- EP-A- 0 289 003
- WO-A-87/04523
- WO-A-91/06559
- DE-A- 3 905 505
- DATABASE WPI Section Ch, Week 9115 Derwent Publications Ltd., London, GB; Class B04, AN 91-105397 XP002084071 & JP 03 046565 A (MITSUI TOATSU CHEM INC) , 27. Februar 1991

## Beschreibung

Die vorliegende Erfindung betrifft ein Verfahren zur immunologischen Bestimmung eines Analyten nach dem Sandwich-Assay-Prinzip und dafür geeignete Bindesubstanzen. Insbesondere betrifft die Erfindung eine Verbesserung derartiger Sandwich-Verfahren, um den Meßbereich zu erweitern und den Hook-Effekt zu verringern.

Die quantitative Bestimmung von antigenen Substanzen mittels Immunassays ist bekannt. Dazu wird häufig ein sogenannter Sandwich-Assay verwendet, wobei zwei gegen gleiche oder verschiedene Epitope des Analyten gerichtete Antikörper mit einer den zu bestimmenden Analyten enthaltenden Probe inkubiert werden. Dabei ist ein erster löslicher Antikörper direkt oder indirekt mit einem signalerzeugenden System, d.h. einer Markierung gekoppelt, während ein zweiter Antikörper (im heterogenen Assay) an eine Festphase gekoppelt vorliegt oder mit einer Bindekomponente versehen ist, wie z.B. Biotin, die zur Bindung mit einer entsprechend beschichteten Festphase fähig ist.

Bei einer Anzahl diagnostisch wichtiger Proteine kann die Konzentration innerhalb eines weiten Bereichs variieren, was bedeutet, daß für die Analytik ein breiter Meßbereich wünschenswert bzw. sogar erforderlich ist. Beispiele derartiger Proteine sind z.B. Krebsparameter, wie etwa α-Fetoprotein (AFP) und Carzinogen-Embryonales Antigen (CEA) sowie auch das Schwangerschaftsprotein Humanes Choriongonadotropin (HCG). Bei der Bestimmung derartiger Analyten ist es einerseits diagnostisch wichtig, einen exakten Wert in hohen Konzentrationsbereichen zu erhalten, um eine erfolgreiche Verlaufskontrolle durchführen zu können, andererseits muß auch im unteren Konzentrationsbereich eine exakte Bestimmung durchführbar sein, um zu einer qualitativ korrekten Diagnose (Ja/Nein) zu gelangen, die ihrerseits grundlegende therapeutische Konsequenzen nach sich ziehen kann.

Ein Problem bei hohen Analytkonzentrationen in einer zu untersuchenden Probe ist der sogenannte "Hook-Effekt", worunter bei sehr hohen Analytkonzentrationen ein Rückgang des nachweisbaren Signals verstanden wird. Der Grund dafür ist darin zu sehen, daß normalerweise in einem heterogenen Sandwich-Assay-Format der lösliche Antikörper und der Festphasenantikörper in Relation zum nachzuweisenden Analyten im Überschuß vorhanden sind, so daß die Sandwich-Komplexe im wesentlichen vollständig ausgebildet und auch detektiert werden können; im Fall einer hohen Analytkonzentration steht jedoch einer begrenzten Anzahl von Antikörpern eine sehr große Anzahl von Analytmolekülen gegenüber. Im Extremfall liegt der Festphasenantikörper im Unterschuß vor, so daß der Analyt nur zum Teil gebunden wird und der Anteil der festphasengebundenen Analyten kann darüber hinaus nicht vollständig detektiert werden, da markierter Antikörper unter Ausbildung löslicher Detektionsantikörper : Analyt-Komplexe durch den Überschuß an Analyt abgefangen wird. Dies hat eine Verringerung des Meßsignals zur Folge, die zu einem falsch negativen Testergebnis führen kann.

Eine Lösung des Problems besteht selbstverständlich darin, die zu untersuchende Probe ausreichend zu verdünnen. Da in der Praxis jedoch nicht von vornherein bekannt ist, wann und wie stark eine Verdünnung erfolgen muß, bedeutet dies, daß mehrere Messungen mit unterschiedlichen Konzentrationen durchgeführt werden müssen, was aus Kostengründen und wegen dem erhöhten Arbeitsaufwand unerwünscht ist.

EP 0 289 003 offenbart ein Verfahren zur quantitativen Bestimmung eines polyvalenten Antigens mit drei verschiedenen Rezeptoren, wobei R1 und R3 in flüssiger Phase gelöst vorliegen und mit dem Antigen bindefähig sind, R2 in fester Phase vorliegt und mit R1 bindefähig ist, und R3 eine Markierung trägt und mit R1 und R2 nicht kreuzreagiert. Demzufolge bindet der Erfindung der Rezeptor R1 mit der Festphase R2 und die Markierung des Antigens erfolgt über den in flüssiger Phase gelösten Rezeptor R3.

DE-A-39 05 505 und WO91/06559 offenbaren oligomere Antikörper zur Verringerung von Störfaktoren, wie unspezifische Reaktionen und Falschpositiv-Ergebnissen. Es werden Antikörper ohne spezifische Reaktivität mit dem zu bestimmenden Antigen verwendet.

Die offenbarten Lösungsansätze enthalten jedoch keinerlei Hinweis auf eine Erweiterung des Meßbereichs in immunologischen Nachweisverfahren.

Eine weitere Lösung dieses Problems besteht in einer Erhöhung der Konzentration der beiden im Sandwich-Assay verwendeten Antikörper. Daraus ergibt sich jedoch eine Reihe von Nachteilen, wie beispielsweise zusätzliche Störungsmöglichkeiten, ein hoher Leerwert und eine ungünstig verlaufende Eichkurve. Zudem erhöhen sich auch die Kosten pro Einzelbestimmung, so daß auch diese Möglichkeit nicht als zufriedenstellend angesehen werden kann.

Eine nochmals weitere Lösung ist in US-Patent 4,743,542 offenbart. Gemäß der Lehre dieses Patents kann eine Linearisierung der Eichkurve bei hohen Analytkonzentrationen erreicht werden durch Zugabe von unmarkiertem ersten oder zweiten Antikörper bzw. von Gemischen davon. Dadurch wird die Empfindlichkeit im unteren Meßbereich gesenkt, bei höheren Analytkonzentrationen jedoch erhöht, so daß insgesamt eine Linearisierung der Eichkurve resultiert.

Ein Nachteil des Verfahrens gemäß US-Patent Nr. 4,743,542 ist jedoch die Verringerung der Empfindlichkeit im unteren Meßbereich. Eine derartige Verringerung ist insbesondere kritisch, wenn ein genaues Testergebnis im unteren Meßbereich essentiell ist, beispielsweise beim HCG-Test, um festzustellen, ob eine Schwangerschaft vorliegt oder nicht.

Die der Erfindung zugrundeliegende Aufgabe bestand somit darin, ein Verfahren zur immunologischen Bestimmung eines Analyten bereitzustellen, bei dem die Nachteile des Standes der Technik mindestens teilweise beseitigt sind und bei dem insbesondere eine quantitativ exakte Bestimmung über einen breiten Meßbereich möglich wird und gleichzeitig im unteren Konzentrationsbereich gute Resultate erhalten werden.

Diese Aufgabe wird erfindungsgemäß gelöst durch ein Verfahren zur immunologischen Bestimmung eines Analyten in einer Probeflüssigkeit nach dem Prinzip des Sandwich-Assays, wobei man die Probeflüssigkeit in Gegenwart einer Festphase mit mindestens zwei mit dem zu bestimmenden Analyten bindefähigen Rezeptoren inkubiert, wobei der erste Rezeptor löslich ist und in markierter Form vorliegt und der zweite Rezeptor (a) an eine Festphase gebunden ist oder (b) mit einer Festphase bindefähig ist, und den Analyten durch Bestimmung der Markierung in der Festphase oder/und in der flüssigen Phase nachweist, welches dadurch gekennzeichnet ist, daß der erste Rezeptor ein Oligomer eines Bindemoleküls ausgewählt aus Antikörpern, Antikörperfragmenten und Gemischen davon ist.

Es hat sich überraschenderweise gezeigt, daß durch die Verwendung eines löslichen oligomeren Antikörpers eine Reduzierung des Hook-Effekts und somit eine Erweiterung des Meßbereichs ermöglicht wird. Dabei wird die Empfindlichkeit im unteren Meßbereich im allgemeinen beibehalten bzw. sogar verbessert. Als Maß für die Empfindlichkeit im unteren Meßbereich wird in der vorliegenden Beschreibung die untere Nachweisgrenze UNG nach Kayser (Fresenius Zeitschrift für analytische Chemie, Band 209, Heft 1, Seite 1-18, 1965) verwendet.

Der erfindungsgemäß verwendete erste Rezeptor ist ein Oligomer eines Bindemoleküls ausgewählt aus Antikörpern oder/und Antikörperfragmenten und wird im folgenden der Einfachheit halber als "oligomerer Antikörper" bezeichnet. Der Zusatz "erster" bzw. "zweiter" Antikörper hat in dieser Beschreibung lediglich eine Unterscheidungsfunktion und kennzeichnet nicht etwa eine Zugabereihenfolge etc. Unter dem Begriff "Antikörper" wird in der vorliegenden Erfindung sowohl ein Antikörper mit einer singulären Spezifität wie etwa ein monoklonaler Antikörper verstanden, als auch ein Gemisch von Antikörpern, die gegen verschiedene Epitope desselben Antigens gerichtet sind, wie etwa ein polyklonales Antiserum. Unter "Antikörperfragment" wird jedes Molekül verstanden, welches von einem kompletten Antikörper unter Beibehaltung mindestens eines Paratops abgeleitet ist und welches erhalten werden kann beispielsweise durch enzymatische oder chemische Behandlung eines Antikörpers oder auf gentechnischem Wege. Insbesondere wird unter einem Antikörperfragment ein F(ab')₂-Fragment verstanden.

Der Oligomerisierungsgrad beträgt, bezogen auf ein vollständiges Antikörper- bzw. ein F(ab')₂-Fragment, mindestens zwei, d.h. die minimale Paratopanzahl eines erfindungsgemäßen oligomeren Antikörpers beträgt vier. Bevorzugt beträgt der minimale Oligomerisierungsgrad zwei bis drei. Der maximale Oligomerisierungsgrad beträgt bis zu 15, bevorzugt bis 10 und stärker bevorzugt bis 8. Am meisten bevorzugt beträgt der Oligomerisierungsgrad 2 bis 8, insbesondere bevorzugt 4 bis 6.

Der oligomere erste Rezeptor wird im erfindungsgemäßen Verfahren vorzugsweise in markierter Form verwendet. Das bedeutet, daß er direkt oder indirekt an eine Markierungsgruppe gekoppelt ist. Unter einer direkten Kopplung wird dabei ein kovalenter Einbau einer nachweisbaren Substanz verstanden oder eines Moleküls, das bei Umsetzen mit einem geeigneten Substrat eine nachweisbare Substanz erzeugt. Ein Beispiel für letzteres wäre z.B. ein Enzym, das kovalent, gegebenenfalls über einen Spacer, an den Rezeptor gebunden ist. Eine indirekte Kopplung bezeichnet eine Konfiguration, wobei der erste Rezeptor gemäß der vorliegenden Erfindung und eine nachweisbare Substanz bzw. ein eine derartige Substanz erzeugendes Molekül über ein spezifisches Bindungspaar, wie etwa Biotin/Avidin, miteinander bindefähig sind.

Die Markierungsgruppe im erfindungsgemäßen Verfahren kann aus im Stand der Technik bekannten Markierungen ausgewählt werden. Beispiele sind Radiomarkierungen sowie Enzymmarkierungen oder Lumineszenzmarkierungen. Bevorzugte Beispiele für Enzymmarkierungen sind z.B. alkalische Phosphatase, Peroxidase und Galactosidase. Als Lumineszenzmarkierungen bevorzugt sind Ca-aktivierbare Photoproteine wie Aequorin und Elektrochemilumineszenzmarkierungen. Markierungen, bei denen die Bestimmung über eine Lumineszenzreaktion verläuft, sind besonders bevorzugt und am meisten bevorzugt ist als Markierungsgruppe eine lumineszierende Metallchelat-Markierungsgruppe.

Die lumineszierenden Metallchelate sind Metallchelate, die eine nachweisbare Lumineszenzreaktion erzeugen. Der Nachweis dieser Lumineszenzreaktion kann beispielsweise durch Fluoreszenz- oder durch Elektrochemilumineszenzmessung erfolgen. Das Metall dieser Metallchelate ist beispielsweise ein Übergangsmetall oder ein Seltenerdmetall. Vorzugsweise ist das Metall Ruthenium, Osmium, Rhenium, Iridium, Rhodium, Platin, Indium, Palladium, Molybdän, Technetium, Kupfer, Chrom oder Wolfram. Besonders bevorzugt ist Ruthenium, Rhenium, Iridium und Osmium und am meisten bevorzugt ist Ruthenium.

Die Liganden, die zusammen mit dem Metall das Metallchelat bilden, sind üblicherweise Polydentat-Liganden, d.h. Liganden mit mehreren Koordinationsstellen. Polydentat-Liganden umfassen beispielsweise aromatische und aliphatische Liganden. Geeignete aromatische Polydentat-Liganden beinhalten aromatische heterocyclische Liganden. Bevorzugte heterocyclische Liganden sind N-haltige Polyheterocyclen wie etwa z.B. Bipyridyl, Bipyrazyl, Terpyridyl und Phenanthrolyl. Diese Liganden können beispielsweise Substituenten wie etwa Alkyl, substituiertes Alkyl, Aryl, substituiertes Aryl, Aralkyl, Carboxylat, Carboxyaldehyd, Carboxamid, Cyano, Amino, Hydroxy, Imino, Hydroxycarbonyl, Aminocarbonyl, Amidin, Guanidinium, Ureid, schwefelhaltige Gruppen, phosphorhaltige Gruppen und die Carboxylatester von N-Hydroxysuccinimid umfassen. Das Chelat kann auch einen oder mehrere Monodentat-Liganden enthalten. Beispiele von Monodentat-Liganden umfassen Kohlenmonoxid, Cyanide, Isocyanide, Halogenide und aliphatische, aromatische und heterocyclische Phosphine, Amine, Stilbene und Arsine.

Besonders bevorzugt wird das lumineszierende Metallchelat ausgewählt aus Metallchelaten mit Bipyridyl- oder Phenanthrolyl-Liganden. Beispiele geeigneter Metallchelate und ihre Herstellung sind in EP-A-0 178 450, EP-A-0 255 534, EP-A 0 580 979 und WO 90/05301 beschrieben. Auf diese Offenbarung wird hiermit Bezug genommen. Am meisten bevorzugte Metallchelate sind Ruthenium-(bipyridyl)₃-Chelate. Diese Chelate sind in Form von Aktivesterderivaten kommerziell erhältlich, z.B. von Igen Inc. (Rockville, MD, USA).

Der im erfindungsgemäßen Verfahren verwendete markierte Rezeptor enthält eine oder mehrere Markierungsgruppen. Falls mehrere Markierungsgruppen pro Molekül des ersten oligomeren Rezeptors eingesetzt werden, ist es bevorzugt, daß die Anzahl der Markierungsgruppen 2 bis 20, stärker bevorzugt 2 bis 10 beträgt.

Der zweite im erfindungsgemäßen Verfahren verwendete Rezeptor ist an die Festphase gebunden oder mit ihr bindefähig. Unter dem Begriff "an die Festphase gebunden" wird in dieser Beschreibung eine zeitlich vor dem Assay stattfindende Immobilisierung durch Anheftung an einen makromolekularen Träger verstanden, wie etwa durch Adsorption, kovalente Bindung oder mittels eines spezifischen Bindepaares. Unter dem Begriff "bindefähig" wird dementsprechend eine Immobilisierung während des Assays mittels der spezifischen Wechselwirkung eines geeigneten spezifischen Bindepaares wie etwa Biotin/Avidin verstanden. Bevorzugt erfolgt die Kopplung des zweiten Rezeptors an die Festphase über ein spezifisches Bindepaar. Besonders bevorzugt ist der zweite Rezeptor biotinyliert und die Festphase mit Avidin oder/und Streptavidin beschichtet.

Die Art des zweiten Rezeptors ist im Verfahren der vorliegenden Erfindung nicht sonderlich kritisch und demgemäß kann der zweite Rezeptor aus einer Anzahl von Molekülen ausgewählt werden, vorausgesetzt, daß er eine spezifische Bindung mit dem zu bestimmenden Analyten eingehen kann. Beispiele von Substanzen, die geeignet als zweiter Rezeptor eingesetzt werden können, sind etwa oligomere Antikörper im Sinne dieser Erfindung, Antikörper, Antikörper-Fragmente, Zellrezeptoren, Enzyme, Nukleinsäuren und andere Substanzen, die aufgrund einer besonderen Raumstruktur oder chemischen Affinität den zu bestimmenden Analyten zu binden vermögen.

Als Festphase werden im erfindungsgemäßen Verfahren in Abhängigkeit von der genauen Ausgestaltung der Versuchsdurchführung herkömmliche Festphasen verwendet. Diese umfassen z.B. feinverteilte Materialien, wie etwa Polystyrolkügelchen oder teilchenförmiges magnetisches Material sowie allgemein Oberflächen von Reaktionsgefäßen, wie etwa die Wand einer Küvette oder einer Mikrotiterplatte, und für besondere Anwendungen Chips, Sensoren oder Membranen. In der derzeit bevorzugten Ausführungsform des erfindungsgemäßen Verfahrens ist die Festphase teilchenförmiges magnetisches Material, das eine mit dem zweiten Rezeptor bindefähige Beschichtung enthält.

Die genaue Durchführung des erfindungsgemäßen Verfahrens entspricht bekannten Protokollen von Sandwich-Assays und ist dem Fachmann geläufig. Allgemein wird eine den zu bestimmenden Analyten enthaltende Probe unter geeigneten Bedingungen und für einen ausreichenden Zeitraum mit erstem Rezeptor, zweitem Rezeptor und Festphase in einer beliebigen Reihenfolge inkubiert, um die Ausbildung eines immobilisierten Sandwich-Komplexes zu gewährleisten. Danach wird der den Analyten enthaltende gebildete Sandwich-Komplex von freiem markiertem Antikörper abgetrennt und, gegebenenfalls nach Zugabe eines geeigneten Nachweissubstrats, qualitativ oder quantitativ bestimmt.

In einer bevorzugten Ausführungsform des erfindungsgemäßen Verfahrens wird die zu untersuchende Probe zunächst mit einem Ruthenium-Chelat-markierten oligomeren Antikörper und einem biotinyliertem Antikörper gemischt und inkubiert. In einem zweiten Schritt werden magnetische Beads, die mit Avidin oder Streptavidin beschichtet sind, zugegeben und weiter inkubiert. Danach wird das Gemisch über einen Magneten geführt, die Beads, d.h. gebildeter Komplex von der restlichen Probe bzw. von nicht-gebundenem markierten Antikörper abgetrennt und der abgetrennte Komplex mit einer Lösung, die ein Reduktionsmittel wie etwa Tripropylamin (TPA) sowie gegebenfalls ein oberflächenaktives Mittel enthält, nochmals gewaschen. Durch den Waschvorgang wird das gesamte Ruthenium bis auf das im Sandwich-Komplex gebundene entfernt. Die gemessene Ru-Konzentration ist somit direkt proportional zur Analyt-Konzentration.

Beim Anlegen einer elektrischen Spannung kommt es zu einer Lumineszenzreaktion, bei der das Ruthenium und das TPA reagieren.

Gegebenenfalls kann bei Verwendung eines markierten ersten Rezeptors zusätzlich ein unmarkierter Analyt-spezifischer Rezeptor vorhanden sein, z.B. ein unmarkierter monomerer Antikörper oder/und oligomerer Antikörper.

Gemäß einem weiteren Aspekt betrifft das Verfahren den Einsatz eines oligomeren Antikörpers umfassend ein Konjugat aus mindestens zwei Antikörpern, Antikörperfragmenten und Gemischen davon, wobei die Bindemoleküle kovalent direkt miteinander verbunden sind und welcher dadurch gekennzeichnet ist, daß der Oligomerisierungsgrad 2 bis 15 beträgt.

Unter einer direkten Verbindung der Bindemoleküle wird verstanden, daß die Antikörper oder Antikörperfragmente entweder über eine einfache chemische Bindung oder über ein geeignetes bifunktionelles Linker-/Spacermolekül miteinander verknüpft sind. Möglichkeiten um dies zu bewerkstelligen und geeignete Spacermoleküle sind dem Fachmann in großer Anzahl bekannt. Als chemische Linker kommen beispielsweise Bis(maleinimido)-methylester, Dimethylsuberimidat, Disuccinimidylsuberat, Glutardialdehyd, N-Succinimidyl-3-(2-pyridyldithio)propionat, N-5-Azido-2-nitrobenzoylsuccinimid, N-Succinimidyl(4-iodacetyl)-aminobenzoat, eine Kombination von Maleinimidohexanoyl-Succinimidat und S-Acetyl-mercaptobernsteinsäureanhydrid, bzw. analoge Verbindungen in Betracht.

In einer bevorzugten Ausführungsform erfolgt die Verwendung des erfindungsgemäßen oligomeren Antikörpers entsprechend dem ersten Aspekt der Erfindung mit mindestens einer und gegebenenfalls mehreren Markierungsgruppen versehen. Bevorzugt ist als Markierung eine lumineszierende Metallchelatmarkierung und besonders bevorzugt ist der oligomere Antikörper ruthenyliert, d.h. das Metallchelat ist ein Rutheniumchelat. Die Liganden, die zusammen mit dem Metall das Metallchelat bilden, sind üblicherweise Polydentatliganden, d.h. Liganden mit mehreren Koordinationsstellen. Derartige Liganden und Metallchelate sind z.B. in DE-A-44 30 998.8 beschrieben, auf deren Offenbarung hiermit Bezug genommen wird. Die am meisten bevorzugten Metallchelate sind Ruthenium (bipyridyl)₃-Chelate.

Ein nochmals weiterer Aspekt der vorliegenden Erfindung betrifft die Verwendung eines oligomeren Antikörpers, umfassend ein Konjugat aus mindestens zwei Antikörpern, Antikörperfragmenten oder/und Gemischen davon, wobei der Oligomerisierungsgrad 2-15 beträgt, und mindestens einer Markierungsgruppe als Nachweisreagenz in einem Sandwich-Assay. Außerdem kann der oligomere Antikörper mit einem Oligomerisierungsgrad von 2-15 zur Verringerung oder/und Vermeidung des Hook-Effekts in einem immunologischen Bestimmungsverfahren eingesetzt werden.

Ein nochmals weiterer Aspekt der vorliegenden Erfindung betrifft einen Reagenzienkit zur immunologischen Bestimmung eines Analyten, der zu der Durchführung des erfindungsgemäßen Verfahrens geeignet ist. Ein derartiger Kit umfaßt insbesondere einen markierten oligomeren Antikörper wie vorstehend definiert, wobei die Markierungsgruppen bevorzugt lumineszierende Metallchelat-Markierungsgruppen sind und deren Anzahl 2-20 beträgt, und den im erfindungsgemäßen Verfahren verwendeten zweiten Rezeptor, also einen Festphasen-gebundenen bzw. bevorzugt mit einer Festphase bindefähigen Rezeptor, z.B. einen Antikörper oder/und ein Antikörperfragment oder/und Gemischen davon. Der oligomere Antikörper und der zweite Rezeptor können im erfindungsgemäßen Reagenzienkit getrennt vorliegen oder gemeinsam in einem einzigen Reagenz enthalten sein. Gegebenenfalls enthält der Kit auch weitere Reagenzien, wie etwa eine geeignete Festphase, eine Substratlösung etc., die dann im allgemeinen räumlich getrennt vorliegen.

Das erfindungsgemäße Verfahren wird durch die nachfolgenden Beispiele näher erläutert.

### BEISPIELE

### Beispiel 1

### Herstellung biotinylierter bzw. oligomerer und markierter Antikörper

### 1. Monomerer biotinylierter Anti-HCG-Antikörper

Monoklonales Anti-HCG-M-1F7.9-IgG (Boehringer Mannheim GmbH) wird in 0,1 M Kalium-Phosphat-Puffer, pH 8,4, zu einer Konzentration von 10 mg/ml gelöst. Man setzt die 6-fach molare Menge an Biotin-DDS* (gelöst in Dimethylsulfoxid; 16,3 mg/ml) hinzu. Nach 90 min Rühren bei 25°C wird der Ansatz durch Zugabe von 10 µl 1 M Lysin gestoppt. Man dialysiert gegen 25 mM Kaliumphosphat/50 mM NaCl, pH 7,0, und lyophilisiert das Produkt.
* Biotin(DDS) = Biotinyl-amino-3,6-dioxaoctanyl-aminocarbonyl heptansäure-N-hydroxysuccinimidester (siehe DE 4302241 A1)

### 2. Oligomerer ruthenylierter Anti-HCG-Antikörper

a) Ruthenium-Markierungsgruppe
   Für die Markierung von Antikörpern und Antikörper-Fragmenten wird der aktive Ester Ru(bpy)₃²⁺-NHS (Ruthenium (2,2'-bipyridyl)₂(4-[3-(N-hydroxysuccinimidylcarboxy)propyl]-4'-methyl-2,2'-bipyridin)²⁺) verwendet, der von der Fa. Igen, Inc., Rockville, USA, bezogen werden kann.
b) Herstellung von Anti-HCG-F(ab')₂-Fragmenten
   300 mg des monoklonalen Anti-HCG-M-INN22-IgG Antikörpers werden nach Johnstone and Thorpe (Immunochemistry in Practice, Seite 61 f, Blackwell Scientific, 1987) mit Pepsin gespalten und die F(ab')₂-Fragmente durch S 200 Chromatographie gereinigt. Ausbeute: 128 mg F(ab')₂.
c) Herstellung und Isolierung von oligomeren Antikörper-Fragmenten
   100 mg Anti-HCG-M-INN22-F(ab')₂-Fragmente werden in 1,5 ml 0,15 M K-Phosphat-Puffer, pH 8,3 gelöst. Kurz vor Gebrauch löst man 27,6 mg Disuccinimidyl-suberat (DSS) in 1 ml Dimethylsulfoxid. Unter Rühren werden in Abständen von 10 min der F(ab')₂-Lösung je 5 µl DSS-Lösung zugesetzt. Nach insgesamt 75 min Umsetzungsdauer wird die Vernetzung des F(ab')₂ mit 15 µl 1 M Lysin-Lösung gestoppt.
   Durch Gelchromatographie an Sephacryl S300HR (Pharmacia, Upsala) wird das Anti-HCG-M-INN22-F(ab')₂-Rohpolymerisat in Fraktionen mit Molekulargewichten von 200.000, 400.000, 500.000-800.000 und > 800.000 aufgetrennt. Die Polymere ≤ 800.000 sind bevorzugt.
d) Herstellung von ruthenylierten oligomeren Antikörper-Fragmenten
   5 mg Anti-HCG-M-INN22-F(ab')₂-Oligomer (Mol. Gew. 400.000) werden in 1 ml 0,15 M K-Phosphat-Puffer, 0,15 M NaCl, pH 7,8 gelöst. Unmittelbar vor Gebrauch löst man 5 mg Ru(bpy)₃²⁺-NHS in 0,75 ml wasserfreiem Dimethylsulfoxid. Um ein Molverhältnis von 3,3:1 zu erhalten, bezogen auf die Molekulargewichte 1057 für Ru(bpy)₃²⁺-NHS und 100.000 für F(ab')₂-Monomer, werden 0,174 mg Ru(bpy)₃²⁺-NHS (59,4 µl) zur F(ab')₂-Lösung unter Rühren zupipettiert.
   Das Reaktionsgefäß wird 60 min. bei 25°C inkubiert. Zum Abstoppen der Umsetzung pipettiert man 10 µl einer 1 M Lysin-Lösung hinzu.
   Der Ansatz wird dann 24 Std. gegen 25 mM K-Phosphatpuffer/0,1 M NaCl, pH 7,0, dialysiert und lyophilisiert.
   Ausbeute: 4,4 mg Anti-HCG-M-INN22-F(ab')₂-Ru(bpy)₃²⁺ Oligomer (400.000).
   Über Extinktionsmessung bei 455 nm (ε=13.7) ergibt sich ein Molverhältnis an eingebauter Markierung von 2,2-2,8=Ru:F(ab')₂.
   Oligomere Antikörperfragmente mit Molekulargewichten von 200.000 bzw. 500.000-800.000 werden auf analoge Weise mit Ruthenium-Markierungsgruppen gekoppelt.

### Beispiel 2

### Nachweis von HCG unter Verwendung oligomerer Antikörper

Die folgenden zwei Lösungen, enthaltend markierten oligomeren ruthenylierten Antikörper bzw. monomeren biotinylierten Antikörper, werden vorbereitet:

### Lösung 1:

Als oligomerer Rezeptor werden monoklonale, Ru(bpy)₃ markierte Anti-HCG-F(ab')₂-Antikörperfragmente verwendet, die gemäß Beispiel 1 erhältlich sind (nachfolgend als AK-BPRU bezeichnet).

AK-BPRU wird mit einem Oligomerisierungsgrad von 5-7 und einem Ruthenylierungsgrad von 3,3 eingesetzt. Zur Herstellung von Lösung 1 werden 3,0 µl/ml AK-BPRU in 40 mmol/l Phosphatpuffer (pH = 6,5) enthaltend 0,1 % Rinderimmunglobulin (IgG), 3,5% Rinderserumalbumin (RSA) und 150 mmol/l NaCl gelöst und gemischt.

### Lösung 2:

Monomere biotinylierte Anti-HCG-Antikörper (IgG) werden in einer Konzentration von 5,0 µg/ml, Biotinylierungsgrad = 1:10 in einem 80 mmol/l Phosphatpuffer (pH = 7,0) enthaltend 0,1% Rinder-IgG, 2,0% RSA und 150 mmol NaCl gelöst.

100 µl Lösung 1 und 100 µl Lösung 2 werden gemischt, mit 25 µl der zu analysierenden Probe (Humanserum) versetzt und das Gemisch 5 min. bei 37°C inkubiert. Danach werden 35 µl einer Suspension von Streptavidin-beschichteten Beads mit Eisenkern, Biotin-Bindekapazität 450 bis 650 ng/ml, Konzentration der Lösung 720 µg/ml zugegeben und weitere 5 min. inkubiert. Anschließend werden die Beads abgetrennt, mit Tripropylamin enthaltender Lösung gewaschen und die Elektrochemilumineszenz bestimmt. Als Beads werden in den Beispielen im allgemeinen Dynabeads M-280 Streptavidin (Dynal A.S., Oslo, Norwegen) verwendet, d.h. superparamagnetische Polystyrol-Partikel mit 2,8 µm Durchmesser, an die Streptavidin kovalent gebunden ist.

Als Ergebnis wird ein Meßbereich bis 12.000 mIU/ml HCG erhalten, wobei eine Probe mit 500.000 mIU/ml HCG als über dem Meßbereich liegend erkannt wird (Hook-Effekt). Die untere Nachweisgrenze ist < 1,0 mIU/ml HCG. Mit Meßbereich wird in dieser Anmeldung der Bereich bezeichnet, in dem die eingesetzten Humanseren unverdünnt korrekt bestimmt werden können.

### Beispiel 3 :

### Einfluß des Oligomerisierungsgrads auf den Meßbereich

Die Versuchsdurchführung erfolgt wie in Beispiel 2 beschrieben, wobei in Lösung 1 jeweils 3,13 µg/ml AK-BPRU eingesetzt werden, die sich in den Oligomerisierungsgraden wie folgt unterscheiden:

| | | |
|---|---|---|
| P1 | MW> | 800.000 |
| P2 | | 500.000 - 800.000 |
| P3 | | 400.000 |
| P4 | | 200.000 |
| P5 | | 100.000 (= F(ab')2 |

Die Ergebnisse sind in Tabelle 1 gezeigt. Es ist zu erkennen, daß mit zunehmendem Vernetzungsgrad (= Molekulargewicht) die Signale der höheren Standards steigen, während der Leerwert (Signal des Null-Standards a) konstant bleibt. Die Sensitivität des Test nimmt somit zu, d.h. mit zunehmendem Oligomerisierungsgrad wird ein breiterer Meßbereich unter Beibehaltung der unteren Nachweisgrenze erhalten.

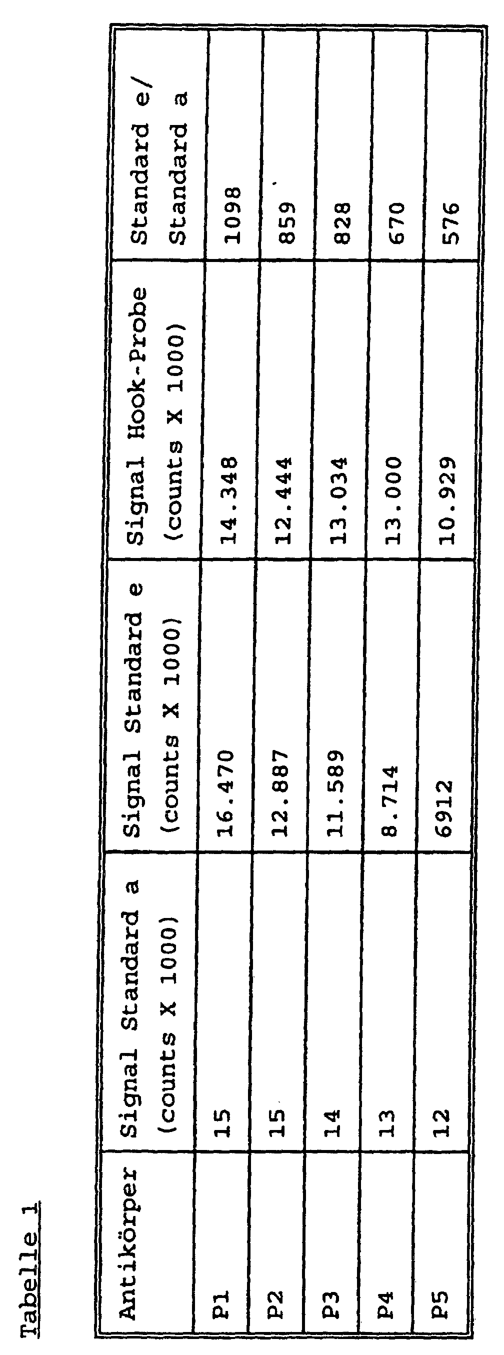

### Beispiel 4:

### Nachweis von HCG mit monomerem monoklonalem Antikörper (Vergleich), Zugabe von unmarkiertem Antikörper

In diesem Beispiel gemäß dem Verfahren von US-Patent 4,743,542 wird in Lösung 1 ein monomerer monoklonaler Antikörper MAK<HCG>-BPRU mit einem Ruthenylierungsgrad von 1:8 in einer Konzentration von 2 µg/ml eingesetzt und der biotinylierte monoklonale Antikörper in Lösung 2 mit einer Konzentration von 2 µg/ml verwendet. Zur Testlösung werden weiterhin unterschiedliche Mengen (2, 4, 8 bzw. 16 µg/ml) eines unmarkierten monomeren monoklonalen Anti-HCG-Antikörpers zugegeben, so daß dieser AK in Konzentrationen von 2, 4, 8 und 16 µg/ml im Testgemisch vorliegt. Es werden 3 Humanseren mit unterschiedlichen HCG-Konzentrationen getestet, HS1 (2941 mIU/ml), HS2 (50.000 mIU/ml) und HS3 (500.000 mIU/ml, Hook-Probe) sowie 2 Standards mit 0,0 (Standard a) und 13,0 mIU/ml (Standard b) an HCG. Die Ergebnisse sind in Tabelle 2 zusammengefaßt.

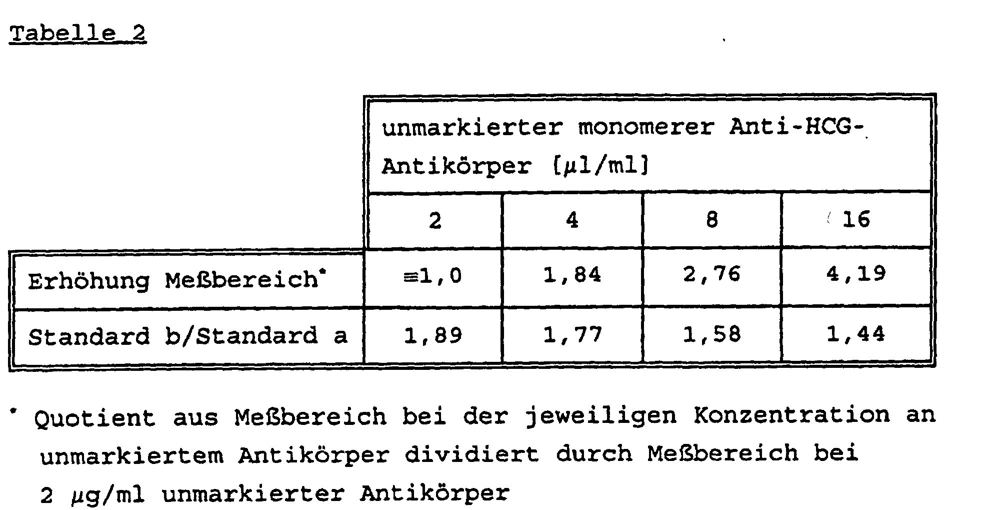

Es wurde gefunden, daß bei zunehmender Menge an unmarkiertem Antikörper der Meßbereich ansteigt. Gleichzeitig nimmt das Signal des Standards b ab, wie auch diejenigen der anderen Humanseren, während das des Standards a (Null-Serum, Leerwert) im wesentlichen konstant bleibt. Hieraus folgt eine steigende Verschlechterung der Differenzierung im unteren Teil des Meßbereichs. Der Quotient Standard b/a, der für eine zuverlässige Aussage mindestens im Bereich von 1,9 bis 2,0 liegen sollte, unterschreitet diesen Wert bereits bei Zusatz von 2 µg/ml unmarkiertem Antikörper. Eine Aussage über eine mögliche Schwangerschaft ist nicht zu machen.

### Beispiel 5:

### Nachweis von HCG mit monomerem polyklonalem Antikörper (Vergleich), Zugabe von unmarkiertem Antikörper

Die Durchführung erfolgt wie bei Beispiel 4, außer daß als unmarkierter Antikörper der polyklonale Antikörper PAK<β-HCG>S-(PA)-IgG(DE) verwendet wurde. Die Ergebnisse entsprechen im wesentlichen dem vom Beispiel 4, der Meßbereich wird jedoch lediglich um Faktor 1,35 erhöht. Eine zuverlässige Unterscheidung im unteren Signalbereich ist nicht möglich. Der Quotient Standard b/a beträgt bei Zusatz von 2 µg/ml des unmarkierten PAK 1,33 und nimmt bei höheren PAK-Konzentrationen weiter ab (16 µg/ml : b/a = 0,94).

### Beispiel 6 :

### Nachweis von HCG mit oligomerem monoklonalem Antikörper Zugabe von unmarkiertem Antikörper

Die Durchführung erfolgt gemäß Beispiel 2, wobei als Detektionsantikörper ein erfindungsgemäßer ruthenylierter oligomerer Antikörper (Oligomerisierungsgrad ca. 4-8) in einer Konzentration von 1, 2 bzw. 4 µg/ml eingesetzt wurde. Die Ergebnisse sind in Tabelle 3 zusammengefaßt.

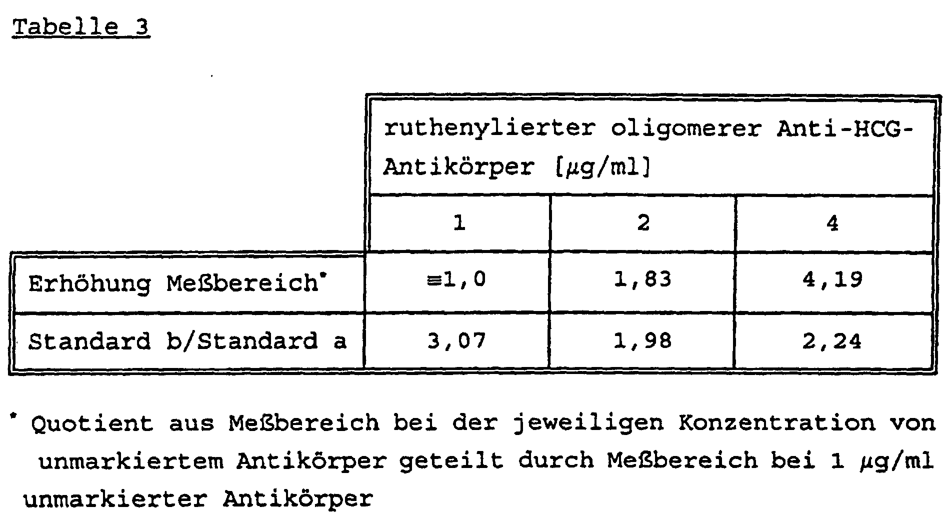

Mit zunehmender AK-BPRU-Konzentration steigt sowohl der Leerwert (Standard a) als auch die Signalhöhe der anderen Proben (Humanseren und Standards). Wie aus der Tabelle ersichtlich, bleibt der Quotient Standard b/a bei bzw. über 2,0 und gewährleistet somit eine gute Messung im unteren Konzentrationsbereich. Gleichzeitig wird der Meßbereich mit zunehmender AK-BPRU-Konzentration beträchtlich erhöht, die im vorliegenden Beispiel sogar nahezu proportional verläuft (eine Verdoppelung der Antikörperkonzentration entspricht in etwa einer Verdoppelung des Meßbereichs).

Da die Erhöhung des Meßbereichs unter Beibehaltung der Meßgenauigkeit im unteren HCG-Konzentrationsbereich stattfindet, liegt hier tatsächlich eine Erweiterung des Meßbereichs in vollem Umfang vor.

### Beispiel 7

### Nachweis von TSH

### Vergleich von oligomerem und monomerem monoklonalem Antikörper

Der TSH-Test dient zur Diagnostik der Schilddrüsenfunktion. Diagnostisch wichtig sind sowohl erhöhte, wie auch stark erniedrigte TSH-Werte im Serum. Aus diesem Grund ist eine hohe Präzision des Tests auch im unteren Bereich wichtig, ebenso eine gute Differenzierung des unteren Standards a zum zweitniedrigsten b. Für den gesamten Meßbereich dient als Kenngröße das Verhältnis e/a, der Quotient des höchsten durch den niedrigsten Standard. Der Quotient sollte möglichst groß sein. In einem ähnlichen System, wie bei HCG beschrieben, wurden folgende Reagentien eingesetzt:
- R1:: Phosphatpuffer, 50 mmol, pH=7,4; jeweils 0,1% MIT und Oxyperoin (zur Konservierung), 2% Rinderserumalbumin, 1% Rinder IgG und ruthenylierter monoklonaler Antikörper MAK <TSH>-m-Fab-BP-Ru in der Konzentration 2,0 µg/ml für das Oligomer und 0,2-1,0 µg/ml für das Monomer.
- R2:: Reagenz wie R1, aber anstelle von MAK-BPRU wird biotinylierter monomerer Antikörper MAK<TSH>F(ab')b-Bi(DDS) in der Konzentration von 1,7 µg/ml eingesetzt.
- Beads:: Es werden Micropartikel mit Eisenkern mit einer Biotinbindekapazität von 700 µg/ml verwendet.

Die Reaktion wird durchgeführt durch Zusammenpipettieren von 70 µl Probe, 80 µl R1 und 80 µl R2, sorgfältiges Mischen und 10 min. Inkubation. Danach werden 50 µl Beads zugegeben und weitere 10 min. inkubiert.

Danach wird das Reaktionsgemisch wie beim HCG beschrieben auf die Elektrode gebracht, die Mikropartikel werden mit Hilfe eines Magneten festgehalten und gewaschen. Es folgt die elektrochemische Reaktion, wobei das Lichtsignal quantitativ gemessen wird.

Das Ergebnis ist in Tabelle 4 dargestellt.

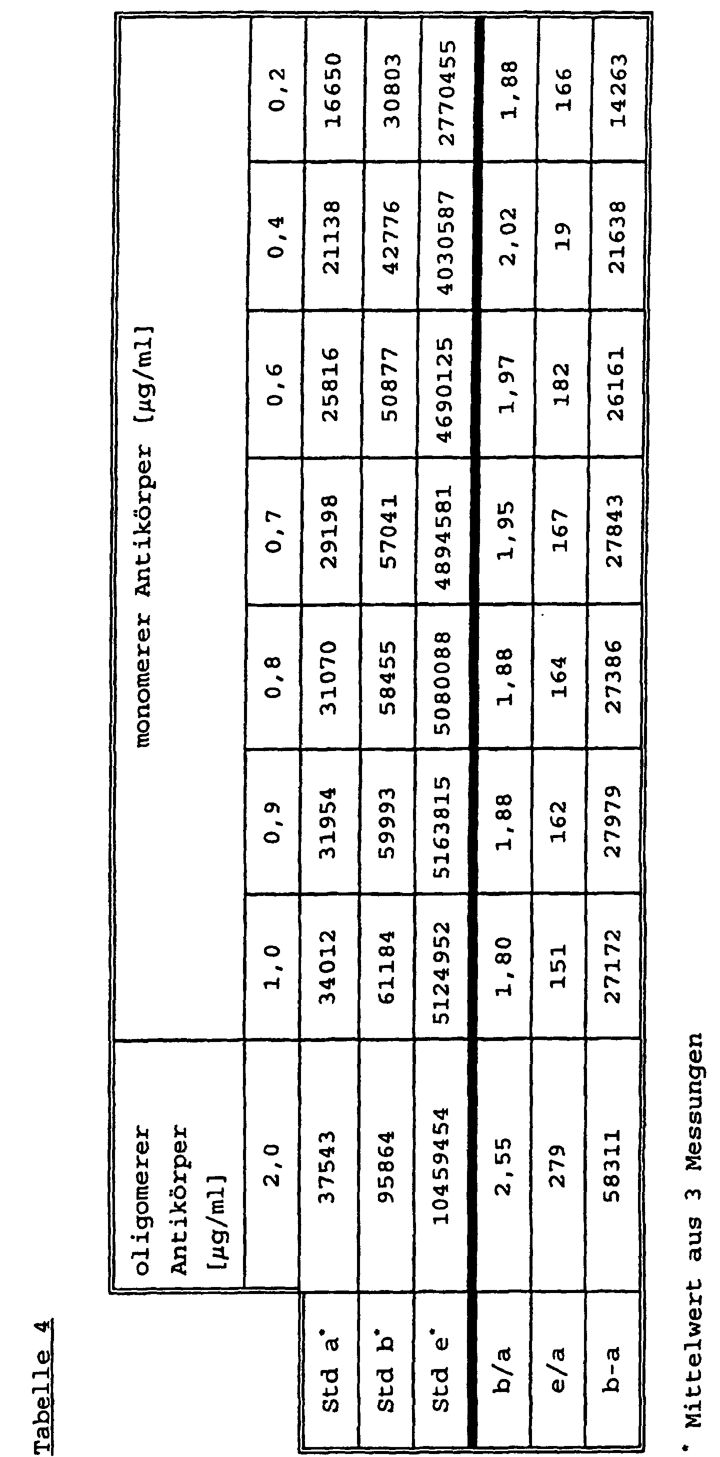

Steigende Konzentrationen an monomerem AK-RU lassen sowohl das Signal des Standards a, wie auch des Standards e ansteigen. Der Quotient b/a bleibt annähernd konstant, eine Verbesserung des Auflösungsverhaltens im unteren Bereich (b-a) wird nicht erreicht, auch die Dynamik des Messbereichs wird nicht verbessert (e/a nahezu konstant). Dieses wird erst durch den Einsatz von oligomerem AK-RU erreicht. Für andere Chargen von Mikropartikeln werden analoge Ergebnisse erhalten.

Durch die Verwendung von oligomeren AK wird die Präzision und Genauigkeit der Messung verbessert, was auch bei Methodenvergleichen sichtbar wird.

### Beispiel 8

### Nachweis von HCG mit oligomerem Antikörper mit/ohne Zugabe von unmarkiertem Antikörper (F(ab')₂)

- R1:: In einem 40 mmol/l Phosphatpuffer, pH=7,5, der außerdem noch 0,1% Rinder IgG und 3% Rinderserumalbumin (RSA) enthält, werden 3,0 µg/ml MAK<HCG>-Ruthenium(II)tris(bipyridyl)-NHS-oligo, als F(ab')₂, Polymerisierungsgrad 5-7, Ruthenylierungsgrad 3,3 gelöst und gemischt.
- R2:: In ein Reagenz wie o.a. wird statt dem MAK-PBRU 5,0 g/ml MAK<HCG>-IgG-biotinyliert, Biotinylierungsgrad 1:10 gegeben.
- R3:: Zu einem Reagenz R1 werden zusätzlich noch 4 µg/ml unmarkierter polyklonaler PAK<HCG>-IgG gegeben und gemischt.

Eine Reihe von HCG-Standards mit Konzentrationen von 0 bis 20.000 mIU/ml werden, wie in Beispiel 7 beschrieben, im Versuchssystem ohne (R1, R2, Beads) bzw. mit Zusatz (R2, R3, Beads) von unmarkiertem monoklonalem Antikörper analysiert.

Es zeigt sich, daß bei Verwendung der Reagenzien R2 und R3 eine flachere Eichkurve erhalten wird als bei Verwendung von R1 und R2. Der Meßbereich ist jedoch im System R2, R3 (Zusatz von unmarkiertem Antikörper) größer.

## Patentansprüche

1. Verfahren zur immunologischen Bestimmung eines Analyten in einer Probeflüssigkeit nach dem Prinzip des Sandwich-Assays, wobei man die Probeflüssigkeit in Gegenwart einer Festphase mit mindestens zwei mit dem zu bestimmenden Analyten bindefähigen Rezeptoren inkubiert, wobei der erste Rezeptor löslich ist und in markierter Form vorliegt und der zweite Rezeptor
(a) an eine Festphase gebunden ist oder
(b) mit einer Festphase bindefähig ist,
und den Analyten durch Bestimmung der Markierung in der Festphase oder/und in der flüssigen Phase nachweist,
**dadurch gekennzeichnet,**
**daß** der erste Rezeptor ein Oligomer eines Bindemoleküls ausgewählt aus Antikörpern, Antikörperfragmenten und Gemischen davon ist.

2. Verfahren nach Anspruch 1,
**dadurch gekennzeichnet,**
**daß** der Oligomerisierungsgrad des ersten Rezeptors 2 bis 15 beträgt.

3. Verfahren nach Anspruch 1 oder 2,
**dadurch gekennzeichnet,**
**daß** der erste Rezeptor 1 bis 2 Markierungsgruppen trägt.

4. Verfahren nach Anspruch 3,
**dadurch gekennzeichnet,**
**daß** die Markierungsgruppe eine lumineszierende Metallchelat-Markierungsgruppe ist.

5. Verfahren nach einem der vorhergehenden Ansprüche,
**dadurch gekennzeichnet,**
**daß** die Festphase aus teilchenförmigem, magnetischem Material besteht und mit dem zweiten Rezeptor oder einem Material, das mit dem zweiten Rezeptor bindefähig ist, beschichtet ist.

6. Verfahren nach einem der vorhergehenden Ansprüchen,
**dadurch gekennzeichnet,**
**daß** man die Bestimmung in Gegenwart eines markierten ersten Rezeptors und zusätzlich eines unmarkierten Analyt-spezifischen Rezeptors durchführt.

7. Verwendung eines oligomeren Antikörpers umfassend ein Konjugat aus mindestens zwei Antikörpern, Antikörperfragmenten oder/und Gemischen davon, wobei der Oligomerisierungsgrad 2-15 beträgt, und mindestens einer Markierungsgruppe als Nachweisreagenz in einem Sandwich-Assay.

8. Verwendung eines oligomeren Antikörpers umfassend ein Konjugat aus mindestens zwei Antikörpern, Antikörperfragmenten oder/und Gemischen davon, wobei der Oligomerisierungsgrad 2-15 beträgt, zur Verringerung oder/und Vermeidung des Hook-Effekts in einem immunologischen Verfahren.

9. Verwendung nach Anspruch 8,
**dadurch gekennzeichnet,**
**daß** der oligomere Antikörper mindestens eine Markierungsgruppe trägt.

10. Reagenzienkit zur immunologischen Bestimmung eines Antigens,
**dadurch gekennzeichnet,**
**daß** er einen oligomeren Antikörper umfassend ein Konjugat aus mindestens zwei Antikörpern, Antikörperfragmenten oder/und Gemischen davon, wobei der Oligomerisierungsgrad 2-15 beträgt, und mindestens eine Markierungsgruppe, und einen an eine Festphase gebundenen oder mit einer Festphase bindefähigen Antikörper umfaßt.

11. Verwendung nach einem der Ansprüche 7 oder 9, bzw. Reagenzienkit nach Anspruch 10,
**dadurch gekennzeichnet,**
**daß** die Markierungsgruppen des oligomeren Antikörpers lumineszierende Metallchelat-Markierungsgruppen sind.

12. Verwendung nach einem der Ansprüche 7 oder 9, bzw. Reagenzienkit nach Anspruch 10,
**dadurch gekennzeichnet,**
**daß** die Anzahl der Markierungsgruppen des oligomeren Antikörpers 2 bis 20 beträgt.

## Claims

1. Method for the immunological determination of an analyte in a sample liquid according to the principle of a sandwich assay in which the sample liquid is incubated in the presence of a solid phase with at least two receptors capable of binding to the analyte to be determined in which the first receptor is soluble and is present in a labelled form and the second receptor
(a) is bound to a solid phase or
(b) is capable of binding to a solid phase
and the analyte is detected by determining the label in the solid phase or/and in the liquid phase,
**wherein**
the first receptor is an oligomer of a binding molecule selected from antibodies, antibody fragments and mixtures thereof.

2. Method as claimed in claim 1,
**wherein**
the degree of oligomerization of the first receptor is 2 to 15.

3. Method as claimed in claim 1 or 2,
**wherein**
the first receptor carries 1 to 2 labelling groups.

4. Method as claimed in claim 3,
**wherein**
the labelling group is a luminescent metal chelate labelling group.

5. Method as claimed in one of the previous claims,
**wherein**
the solid phase is composed of a particulate, magnetic material and is coated with the second receptor or a material which is capable of binding to the second receptor.

6. Method as claimed in one of the previous claims,
**wherein**
the determination is carried out in the presence of a labelled first receptor and additionally of an unlabelled analyte-specific receptor.

7. Use of an oligomeric antibody comprising a conjugate of at least two antibodies, antibody fragments or/and mixtures thereof, wherein the degree of oligomerization is 2-15, and at least one labelling group as a detection reagent in a sandwich assay.

8. Use of an oligomeric antibody comprising a conjugate of at least two antibodies, antibody fragments or/and mixtures thereof, wherein the degree of oligomerization is 2-15 for the reduction or/and avoidance of the Hook effect in an immunological method.

9. Use as claimed in claim 8,
**wherein**
the oligomeric antibody carries at least one labelling group.

10. Reagent kit for the immunological determination of an antigen,
**wherein**
it comprises an oligomeric antibody comprising a conjugate of at least two antibodies, antibody fragments or/and mixtures thereof, wherein the degree of oligomerization is 2-15, and at least one labelling group and an antibody that is bound to a solid phase or is capable of binding to a solid phase.

11. Use as claimed in one of the claims 7 or 9, or a reagent kit as claimed in claim 10,
**wherein**
the labelling groups of the oligomeric antibody are luminescent metal chelate labelling groups.

12. Use as claimed in one of the claims 7 or 9, or a reagent kit as claimed in claim 10,
**wherein**
the number of labelling groups of the oligomeric antibody is 2 to 20.

## Revendications

1. Procédé de dosage immunologique d'un analyte dans un liquide d'essai selon le principe du dosage en sandwich, dans lequel on fait incuber le liquide d'essai en présence d'une phase solide avec au moins deux récepteurs capables de se fixer sur l'analyte à doser, sachant que le premier récepteur est soluble et qu'il est présent sous forme marquée et que le deuxième récepteur
(a) est fixé sur une phase solide ou
(b) est capable de se fixer sur une phase solide,
et permet de mettre l'analyte en évidence dans la phase solide ou/et dans la phase liquide par dosage du marquage,
**caractérisé en ce que**,
le premier récepteur est un oligomère d'une molécule de fixation sélectionnée parmi des anticorps, des fragments d'anticorps et leurs mélanges.

2. Procédé selon la revendication 1,
**caractérisé en ce que**
le degré d'oligomérisation du premier récepteur est compris entre 2 et 15.

3. Procédé selon la revendication 1 ou 2,
**caractérisé en ce que**
le premier récepteur porte 1 à 2 groupes de marquage.

4. Procédé selon la revendication 3,
**caractérisé en ce que**
le groupe de marquage et un groupe de marquage métal chélate luminescent.

5. Procédé selon l'une des revendications précédentes,
**caractérisé en ce que**
la phase solide se compose d'un matériau magnétique sous forme de particules sur laquelle est appliqué en couche le deuxième récepteur ou un matériau capable de se fixer sur le deuxième récepteur.

6. Procédé selon l'une des revendications précédentes,
**caractérisé en ce que**
l'on réalise le dosage en présence d'un premier récepteur marqué et, en plus, en présence d'un récepteur non marqué spécifique à l'analyte.

7. Mise en oeuvre d'un anticorps oligomère comportant un conjugué d'au moins deux anticorps, fragments d'anticorps ou/et leurs mélanges, sachant que le degré d'oligomérisation est compris entre 2 et 15, et d'au moins un groupe de marquage en tant que réactif de mise en évidence dans un dosage en sandwich.

8. Mise en oeuvre d'un anticorps oligomère comportant un conjugué d'au moins deux anticorps, fragments d'anticorps ou/et leurs mélanges, sachant que le degré d'oligomérisation est compris entre 2 et 15, afin de réduire ou/et d'éviter l'effet crochet (hook effect) dans un procédé immunologique.

9. Mise en oeuvre selon la revendication 8,
**caractérisée en ce que**
l'anticorps oligomère porte au moins un groupe de marquage.

10. Kit de réactifs pour dosage immunologique d'un antigène
**caractérisé en qu'**il
comporte un anticorps oligomère comprenant un conjugué d'au moins deux anticorps, fragments d'anticorps ou/et leurs mélanges, sachant que le degré d'oligomérisation est compris entre 2 et 15, et au moins un groupe de marquage et un anticorps fixé sur une phase solide ou capable de se fixer sur une phase solide.

11. Mise en oeuvre selon l'une des revendications 7 ou 9, ou bien kit de réactifs selon la revendication 10
**caractérisé en ce que**
les groupes de marquage de l'anticorps oligomère sont des groupes de marquage métal chélate luminescents.

12. Mise en oeuvre selon l'une des revendications 7 ou 9, ou bien kit de réactifs selon la revendication 10
**caractérisé en ce que**
le nombre de groupes de marquage de l'anticorps oligomère est compris entre 2 et 20.
